(19) Europäisches Patentamt
European Patent Office
Office européen des brevets

(11) Numéro de publication : 0 357 103 B1

(12) # FASCICULE DE BREVET EUROPEEN

(45) Date de publication du fascicule du brevet : **30.09.92 Bulletin 92/40**

(51) Int. Cl.$^5$ : **C07D 213/61**

(21) Numéro de dépôt : **89201906.8**

(22) Date de dépôt : **14.07.89**

(54) **Procédé pour la préparation de 2,6-dichloropyridine et utilisation de la bis(trichlorométhyl)sulfone dans ce procédé.**

Le dossier contient des informations techniques présentées postérieurement au dépôt de la demande et ne figurant pas dans leprésent fascicule.

(30) Priorité : **15.07.88 FR 8809718**

(43) Date de publication de la demande : **07.03.90 Bulletin 90/10**

(45) Mention de la délivrance du brevet : **30.09.92 Bulletin 92/40**

(84) Etats contractants désignés : **AT BE CH DE ES FR GB GR IT LI LU NL SE**

(56) Documents cités :
**EP-A- 0 140 438**
**EP-A- 0 172 595**
**US-A- 3 251 848**

(56) Documents cités :
**REC. TRAV. CHIM., vol. 90, no. 71971, 1971, pages 700-704, Koninklijke Nederlandse Chemische Vereniging; W. DORREPAAL et al.:"Induced vapour phase chlorination of arenes at about 200 degrees Celsius"**

(73) Titulaire : **SOLVAY**
**Rue du Prince Albert, 33**
**B-1050 Bruxelles (BE)**

(72) Inventeur : **Franklin, James**
**Rue Edouard Olivier, 28**
**B-1170 Bruxelles (BE)**
Inventeur : **Janssens, Francine**
**Koningslosteenweg, 106**
**B-1800 Vilvoorde (BE)**

(74) Mandataire : **Nichels, William et al**
**Solvay Département de la Propriété Industrielle Rue de Ransbeek, 310**
**B-1120 Bruxelles (BE)**

EP 0 357 103 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de dérivés chlorés de composés pyridiniques, tels que la 2,6-dichloropyridine, à partir des composés pyridiniques correspondants au moyen de chlore moléculaire en phase gazeuse en présence de la bis(trichlorométhyl)sulfone comme initiateur radicalaire. La présente invention concerne également l'utilisation de la bis(trichlorométhyl)sulfone comme initiateur radicalaire.

A ce jour, différentes techniques ont été développées pour la préparation de dérivés chlorés des composés pyridiniques; une de ces techniques consiste plus particulièrement dans la préparation de la 2,6-dichloropyridine par une réaction de chloration de la pyridine au moyen de chlore moléculaire. Ces techniques ont fait l'objet de mises au point de procédés pouvant être répartis en trois grandes classes.

Une première classe comprend les procédés dits thermiques, effectués en phase vapeur à haute température, généralement supérieure à 350°C, et décrits notamment dans le brevet Etats-Unis 3251848 au nom de DOW. Ces procédés présentent divers désavantages tels que les très hautes températures nécessaires et une formation importante de goudrons, ce qui engendre des bouchages du réacteur ou des conduites, et rend dès lors difficile l'exécution du procédé en continu.

Une deuxième classe de procédés comprend les procédés initiés au moyen de rayonnements lumineux ou ultraviolets. De tels procédés ont été notamment décrits dans le document Chemical Abstract CA 84:164626u. Ces procédés, bien que permettant d'opérer à des températures plus basses que les procédés dits thermiques, présentent l'inconvénient de conduire à la formation de sous-produits goudronneux qui salissent les tubes lumineux et donnent lieu à une diminution subséquente du rayonnement d'initiation et dès lors du rendement de la réaction. En outre, ces procédés ne permettent pas un travail à pression élevée et nécessitent d'opérer en réacteurs perméables aux rayonnements d'initiation, c'est-à-dire le plus généralement en verre, ce qui engendre des difficultés technologiques importantes lorsque les réactions sont extrapolées dans de grandes installations industrielles.

Une troisième classe de procédés comprend les procédés, dits chimiques, catalysés par des initiateurs radicalaires, tels qu'illustrés par la préparation de la 2-chloropyridine notamment dans la demande de brevet EP 0140438 au nom de SOLVAY & Cie dans laquelle les initiateurs radicalaires mis en oeuvre sont des peroxydes organiques tels que le peroxyde de di-tertiaire butyle.

D'autre part, il est connu d'effectuer la chloration d'arènes en présence de bis(trichlorométhyl)sulfone avec une sélectivité élevée en dérivés monochlorés. (Dorrepaal W. et Louw R., Rec. Trav. Chim. (1971), 90, p. 700-704).

La présente invention a pour but de remédier aux inconvénients des procédés connus. En particulier, le procédé selon l'invention conduit à une sélectivité élevée en 2,6-dichloropyridine et ceci à des relativement basses températures. Le procédé conduit également pour une température donnée à une productivité supérieure à celle obtenue avec les procédés connus.

Le procédé pour une température donnée selon l'invention présente un avantage supplémentaire par rapport aux procédés connus; en effet, la séparation des sous-produits d'initiation et co-produits d'avec les composés pyridiniques est aisée et ne pose aucun problème technique.

L'invention concerne à cet effet un procédé pour la préparation de la 2,6-dichloropyridine par une réaction de chloration substitutive de la pyridine au moyen de chlore moléculaire en phase gazeuse et en présence d'un initiateur radicalaire, dans lequel l'initiateur radicalaire est la bis(trichlorométhyl)sulfone mise en oeuvre à raison de 0,01 à 10 % molaire par rapport à la pyridine.

Par réaction de chloration effectuée en phase vapeur, on entend définir une réaction de chloration réalisée dans des conditions de température et de pression telles que tous les réactifs, additifs, catalyseurs et produits issus de la réaction se trouvent à l'état vapeur dans le milieu réactionnel.

Lorsqu'on opère à pression atmosphérique, ces conditions sont remplies pour des températures supérieures à 175°C. De bons résultats ont été obtenus à des températures situées entre 200 et 300°C et, plus particulièrement, entre 220 et 275°C.

La quantité de bis(trichlorométhyl)sulfone mise en oeuvre dans le procédé selon l'invention est généralement faible par rapport aux initiateurs connus. On met en oeuvre de 0,01 à 10 % molaire de bis(trichlorométhyl)sulfone calculé par rapport à la pyridine mise en oeuvre. Les quantités préférées se situent entre 0,1 et 7,5 % molaire par rapport à la pyridine et les quantités particulièrement préférées se situent entre 0,7 et 6 % molaire par rapport à la pyridine.

Le chlore moléculaire et la pyridine sont habituellement mis en oeuvre dans des rapports molaires compris entre 0,1 et 25 moles de chlore par mole de pyridine. De préférence, on met en oeuvre de 0,5 à 15 moles de chlore par mole de pyridine et, de manière particulièrement préférée, 0,7 à 5 moles de chlore par mole de pyridine.

Outre les réactifs et initiateurs déjà cités, on peut mettre en oeuvre dans le procédé selon l'invention des

additifs ou des diluants tels que de la vapeur d'eau, de l'azote et/ou d'autres gaz ne participant pas à la réaction de chloration proprement dite ou des initiateurs intervenant dans la réaction de chloration. Comme additifs, on utilise de préférence des dérivés halogénés de composés aliphatiques tels que le tétrachlorure de carbone ou des produits inorganiques tels que le chlorure d'hydrogène, l'azote ou la vapeur d'eau.

De préférence, on opère avec le tétrachlorure de carbone. En général, les diluants organiques halogénés sont ajoutés au milieu réactionnel à raison de 1 à 25 moles par mole de composé pyridinique mis en oeuvre. Lorsque l'on utilise du tétrachlorure de carbone, comme additif organique, les quantités préférées se situent entre 1,5 et 15 moles par mole de composé pyridinique mis en oeuvre.

La pression à laquelle est réalisé le procédé selon l'invention est habituellement comprise entre 1 et 10 bar, de bons résultats ont été obtenus à pression atmosphérique.

Le temps de séjour des réactifs dans le réacteur est généralement compris entre 1 et 30 s et de préférence entre 2 et 20 s.

Le procédé selon l'invention peut être réalisé dans tout appareillage ou tout réacteur permettant de réunir les conditions opératoires décrites ci-avant.

La 2,6-dichloropyridine obtenue selon le procédé de l'invention peut être utilisée dans toutes les applications connues de ce produit, c'est-à-dire comme intermédiaire chimique pour la fabrication notamment de produits destinés à l'agriculture, de cosmétiques et de produits pharmaceutiques.

Les exemples qui suivent servent à illustrer l'invention.

Exemple 1

La chloration de la pyridine en phase gazeuse a été effectuée à 250°C à la pression atmosphérique dans un réacteur mélangeur continu sphérique d'environ 1 $dm^3$, en pyrex, auto-agité par jets gazeux (Chem. Eng. Sci., 1973, 28, p. 129-137), les réactifs étant introduits sous forme gazeuse à l'aide d'un injecteur à quatre tuyères placé au milieu de la sphère.

Le réacteur est placé dans une enceinte à l'intérieur de laquelle l'air est chauffé électriquement et brassé à l'aide d'une turbine, afin de maintenir la température réactionnelle désirée. Le mélange pyridine-eau est alimenté via un évaporateur tubulaire vertical, chauffé électriquement. Le tétrachlorure de carbone ($CCl_4$) est alimenté via un deuxième évaporateur identique. Le chlore gazeux est injecté dans le pied de l'évaporateur à $CCl_4$. L'initiateur est ajouté sous forme liquide, en solution dans du $CCl_4$, par une dérivation donnant dans le conduit d'introduction du mélange gazeux $CCl_4 + Cl_2$. Les produits de chloration sortent du réacteur par une tubulure diamétralement opposée à l'introduction, puis ils sont condensés, traités par du NaOH aqueux pour neutraliser le chlore résiduaire et le HCl formé. Après décantation, la phase organique est séparée de la phase gazeuse; celle-ci est soumise à une extraction au moyen de chloroforme et la phase organique ainsi que l'extrait chloroformique sont analysés par chromatographie en phase vapeur. L'azote total dans la phase aqueuse extraite est déterminé par la méthode de Kjeldahl.

On met en oeuvre dans ce réacteur 2 moles de tétrachlorure de carbone, 0,05 mole de bis(trichlorométhyl)sulfone, 1 mole de pyridine et 1 mole d'eau. En outre, on injecte en continu 4 moles de chlore moléculaire gazeux. On opère à la pression atmosphérique. Après la mise en régime, on recueille en continu un mélange de liquide ayant la composition suivante (par mole de pyridine mise en oeuvre) :

| | |
|---|---|
| - monochloropyridines | : 0,06 mole |
| - dichloropyridines (hors 2,6-dichloropyridine) | : 0,01 mole |
| - trichloropyridines | : 0,15 mole |
| - 2,6-dichloropyridine | : 0,78 mole |
| - pyridine | : <0,005 mole. |

Le taux de conversion de la pyridine est proche de 100%.

Le temps de séjour des réactifs dans le réacteur est de 5 s; la sélectivité en 2,6-dichloropyridine est de 78 % molaire.

Exemple 2R

On opère comme décrit à l'exemple 1 mais on utilise comme initiateur du peroxyde de di-t-butyle au lieu

et place du bis(trichlorométhyl)sulfone; tous les autres paramètres et conditions sont identiques à ceux décrits à l'exemple 1.

Les résultats sont les suivants :

- monochloropyridines : 0,58 mole
- dichloropyridines (hors 2,6-dichloropyridine) : 0,01 mole
- trichloropyridines : 0,01 mole
- 2,6-dichloropyridine : 0,10 mole
- pyridine : 0,30 mole.

Le taux de conversion de la pyridine est de 70%.

Le temps de séjour des réactifs dans le réacteur est de 10 s; la sélectivité en 2,6-dichloropyridine est de 14 % molaire.

Exemple 3R

On opère comme décrit à l'exemple 1, mais sans initiateur, et à 270°C.

Les résultats sont les suivants :

- monochloropyridines : 0,27 mole
- dichloropyridines (hors 2,6-dichloropyridine) : 0,01 mole
- trichloropyridines : 0,01 mole
- 2,6-dichloropyridine : 0,06 mole
- pyridine : 0,65 mole.

Le taux de conversion de la pyridine est de 35%.

Le temps de séjour des réactifs dans le réacteur est de 10 s; la sélectivité en 2,6-dichloropyridine est de 17 % molaire.

Exemples 4 et 5

On opère comme décrit à l'exemple 1, mais avec des quantités d'initiateurs tels que repris au tableau 1. Les résultats observés sont également repris au tableau 1.

Tableau 1

| | Exemple 4 | Exemple 5 |
|---|---|---|
| **Initiateur** | | |
| bis(trichlorométhyl)sulfone | | |
| mole par mole de pyridine | 0,02 | 0,01 |
| **Résultats** | | |
| - Produits recueillis mole/mole de pyridine mise en oeuvre | | |
| monochloropyridines | 0,07 | 0,15 |
| dichloropyridines (hors 2,6-dichloropyridine) | 0,01 | 0,02 |
| trichloropyridines | 0,11 | 0,06 |
| 2,6-dichloropyridine | 0,81 | 0,76 |
| pyridine | 0 | 0,01 |
| - Bilan | | |
| Temps de séjour seconde | 5 | 5 |
| Sélectivité en 2,6-dichloropyridine % molaire | 80 | 77 |
| Taux de conversion de la pyridine, % | 100 | 99 |

**Revendications**

1 - Procédé pour la préparation de 2,6-dichloropyridine par chloration substitutive de la pyridine au moyen de chlore moléculaire en présence d'un initiateur radicalaire en phase gazeuse, caractérisé en ce que l'initiateur radicalaire est la bis(trichlorométhyl)sulfone mise en oeuvre à raison de 0,01 à 10 % molaire par rapport à la pyridine.

2 - Procédé selon la revendication 1, caractérisé en ce que la bis(trichlorométhyl)sulfone est mise en oeuvre à raison de 0,1 à 7,5 % molaire par rapport à la pyridine.

3 - Procédé selon l'une quelconque des revendications 1 ou 2, caractérisé en ce que la chloration est effectuée à pression atmosphérique à une température supérieure à 175 °C.

4 - Procédé selon la revendication 3, caractérisé en ce que la température est comprise entre 200 et 300 °C.

5 - Procédé selon l'une quelconque des revendications 1 à 4, caractérisé en ce que le temps de séjour des réactifs dans le réacteur de chloration est compris entre 1 et 30 sec.

6 - Procédé selon l'une quelconque des revendications 1 à 5, caractérisé en ce qu'on met en oeuvre le chlore dans un rapport molaire initial compris entre 0,5 à 15 moles de chlore par mole de pyridine.

7 - Procédé selon l'une quelconque des revendications 1 à 6, caractérisé en ce qu'on réalise la réaction de chloration en présence de tétrachlorure de carbone.

8 - Utilisation de la bis(trichlorométhyl)sulfone comme initiateur radicalaire de la chloration substitutive de la pyridine en 2,6-dichloropyridine en phase gazeuse au moyen de chlore moléculaire.

**Patentansprüche**

1. Verfahren zur Herstellung von 2,6-Dichlorpyridin durch substitutive Chlorierung des Pyridins mittels molekularem Chlor in Anwesenheit eines radikalartigen Inititators in gasförmiger Phase, dadurch gekennzeichnet, daß der radikalartige Initiator das bis(Trichlormethyl)sulphon ist, das in einer Menge von 0,01 bis 10 Mol.-% bezogen auf das Pyridin eingesetzt wird.

2. Verfahren nach Anspruch 1, dadurch gekennzeichnet, daß das bis(Trichlormethyl)sulphon in einer Menge von 0,1 bis 7,5 Mol.-% bezogen auf das Pyridin eingesetzt wird.

3. Verfahren nach einem der Ansprüche 1 oder 2, dadurch gekennzeichnet, daß die Chlorierung bei atmosphärischem Druck bei einer Temperatur größer als 175°C durchgeführt wird.

4. Verfahren nach Anspruch 3, dadurch gekennzeichnet, daß die Temperatur zwischen 200 und 300°C liegt.

5. Verfahren nach einem der Ansprüche 1 bis 4, dadurch gekennzeichnet, daß die Aufenthaltsdauer der Reagenzien in dem Chlorierungsreaktor zwischen 1 und 30 Sekunden beträgt.

6. Verfahren nach einem der Ansprüche 1 bis 5, dadurch gekennzeichnet, daß das Chlor in einem molaren Anfangsverhältnis von 0,5 bis 15 Mol Chlor pro Mol Pyridin eingesetzt wird.

7. Verfahren nach einem der Ansprüche 1 bis 6, dadurch gekennzeichnet, daß die Chlorierungsreaktion in Anwesenheit von Tetrachlorkohlenstoff durchgeführt wird.

8. Verwendung des bis(Trichlormethyl)sulphons als radikalartigen Initiator bei der substitutiven Chlorierung des Pyridins zu 2,6-Dichlorpyridin in gasförmiger Phase mittels molekularem Chlor.

**Claims**

1. Process for the preparation of 2,6-dichloropyridine by substitutive chlorination of pyridine using molecular chlorine in the presence of a radical initiator in the gas phase, characterised in that the radical initiator is bis(trichloromethyl) sulphone used in a proportion of 0.01 to 10 mol% relative to pyridine.

2. Process according to Claim 1, characterised in that bis(trichloromethyl) sulphone is used in a proportion of 0.1 to 7.5 mol% relative to pyridine.

3. Process according to either of Claims 1 and 2, characterised in that the chlorination is carried out at atmospheric pressure at a temperature above 175°C.

4. Process according to Claim 3, characterised in that the temperature is between 200 and 300°C.

5. Process according to any one of Claims 1 to 4, characterised in that the residence time of the reactants in the chlorination reactor is between 1 and 30 s.

6. Process according to any one of Claims 1 to 5, characterised in that chlorine is used in an initial molar ratio of between 0.5 to 15 moles of chlorine per mole of pyridine.

7. Process according to any one of Claims 1 to 6, characterised in that the chlorination reaction is carried out in the presence of carbon tetrachloride.

8. Use of bis(trichloromethyl) sulphone as radical initiator of substitutive chlorination of pyridine to 2,6-dichloropyridine in the gas phase by means of molecular chlorine.